# EUROPEAN PATENT SPECIFICATION

(11) **EP 4 124 287 B1**
(45) Date of publication and mention of the grant of the patent: **17.09.2025**
(21) Application number: 22187011.6
(22) Date of filing: 26.07.2022
(51) Int. Cl.: A61B 5/00, A61B 5/02, A61B 5/08, A61B 5/318, A61B 5/369, A61B 5/389

(54) **REGULARIZED MULTIPLE-INPUT PAIN ASSESSMENT AND TREND**
REGULARISIERTE SCHMERZBEWERTUNG MIT MEHRFACHEINGABE UND TREND
ÉVALUATION ET TENDANCE DE LA DOULEUR À INTRANTS MULTIPLES RÉGULARISÉS

(30) Priority: 26.07.2021 US 202163225933 P
(43) Date of publication of application: 01.02.2023
(73) Proprietor: Welch Allyn, INC., Skaneateles Falls, NY 13153-0220 (US)
(72) Inventor: KELLNER, David L, Skaneateles Falls, NY 13153-0220 (US); LANE, John A, Skaneateles Falls, NY 13153-0220 (US)
(74) Representative: Reddie & Grose LLP

(56) References cited:
- US-A1- 2019 313 966
- US-A1- 2020 359 914
- US-A1- 2020 359 960

## Description

Patient care commonly requires clinicians to assess the severity of pain exhibited by patients. Since high levels of pain and prolonged periods of pain may place patients in prolonged distress, hindering recovery, clinicians routinely perform pain management as part of patient care. Pain management entails clinicians assessing intensity of patient pain, and, in response to assessments, providing treatment, intervention, and the like upon the assessed patient. Pain management may be separate from treatments of underlying illness, and pain management may be just as important as other treatments for ongoing patient wellbeing.

Pain assessment is a highly subjective clinician practice, since patients may experience pain reflecting a combination of physiological and psychological factors, where psychological factors in particular cannot be directly measured by instruments or testing. Clinicians have developed various lists of questions which solicit patients to provide personal reports of pain being experienced. By observing patients during care and considering patient answers reporting pain, clinicians may translate a subjective patient reporting of pain into a clinical assessment of pain. Consequently, multiple layers of subjective human judgment may confound the ultimate clinical assessment and result in inconsistent assessments.

Moreover, due to the subjectivity of human judgment, each individual pain assessment over the course of care may be made inconsistent by various confounding factors inherent to human behavior. While clinicians have developed various scales, manually administered tests, and mental guidelines to assist in predictably assessing patient pain, evaluations may unpredictably be confounded by idiosyncratic or willfully misleading patient behavior and responses; limited monitoring and access to a patient over the course of care; differences in a clinician's human judgement at different times; and lack of uniformity in measurement, data collection, responses, and the like during each instance of assessment.

Pain assessments performed in inconsistent fashions may lead to clinicians prescribing and/or administering disproportionate pain management relative to actual pain intensity. Pain management low in proportion to actual pain intensity may hinder patient recovery by failing to alleviate long-term distress, while pain management high in proportion to actual pain intensity may hinder patient recovery in various ways. Thus, clinicians have a need for improved pain assessment tools.

US 2020/0359960 A describes a system for managing pain that includes a pain monitoring circuit. The pain monitoring circuit includes a parameter analyzer and a pain score generator. The parameter analyzer is configured to receive and analyze at least two parameters selected from a physiological parameter indicative of a physiological function or state of a patient, a functional parameter indicative of a physical activity or state of the patient, or a patient parameter including subjective information provided by the patient. The pain score generator is configured to compute a composite pain score using an outcome of the analysis. The composite pain score indicates a degree of the pain. Additional parameters such as environmental parameters (such as temperature, humidity, and/or air pressure) and time can be used in computing the composite pain score. Such environmental parameters can be measured by the system and/or obtained from weather forecasts based on location to anticipate or predict their impact to the composite pain score. One or more weighting factors can be determined based on the reliability of these environmental parameters and applied in computing the composite pain score.

The present invention is defined by the appended independent claims.

The invention will now be further described by way of example with reference to the accompanying drawings, in which:
FIG. 1 illustrates a pain assessment regularizing system according to example embodiments of the present disclosure.
FIG. 2 illustrates a flowchart of a pain assessment factor regularizing method according to example embodiments of the present disclosure.
FIG. 3 illustrates an example system for implementing the processes and methods described herein for implementing regularizing pain assessment factors.

Systems and methods discussed herein are directed to implementing clinical pain assessment, and more specifically capturing pain assessment factor sets from multiple inputs including sensors, and performing, over the course of a patient care program, weighing of different pain assessment factors to derive a regularized pain assessment trend.

According to example embodiments of the present disclosure, a patient care program may span a period of time starting from the patient being placed under a clinician's care, without limitation as to end. The admission to care may be any of various adverse clinical visit or clinical care events, such as a physical checkup, an emergency room visit, a medical procedure such as surgery, and the like. At the time of admission to care, the clinician may diagnose a patient with one or more medical conditions and admit the patient to care in order to monitor the condition, treat the condition, facilitate recovery, and the like; may admit the patient care in order to enable ongoing observation, care, and recovery in order to diagnose one or more unknown medical conditions; the patient may suffer from one or more medical conditions as a result of a medical procedure, requiring ongoing observation, care, and/or recovery while admitted; and the like.

In each such example, the patient may exhibit and/or report experiencing pain on a persistent, long-term, recurring, or otherwise lasting basis; the clinician may determine that the patient is experiencing pain which is likely to occur on a lasting basis; or a medical procedure performed on the patient may cause a medical condition which is causing, or is likely to cause, pain occurring on a lasting basis.

According to example embodiments of the present disclosure, over the course of a patient care program, a clinician may perform a number of pain assessments upon a patient. These may include a first impression pain assessment, performed before the clinician has established a pain assessment trend for the patient; and may include subsequent pain assessments, performed after the clinician has established a pain assessment trend for the patient. A clinician performing at least one pain assessment may cause the clinician to operate a pain assessment regularizing system to establish a pain assessment trend, as shall be described subsequently.

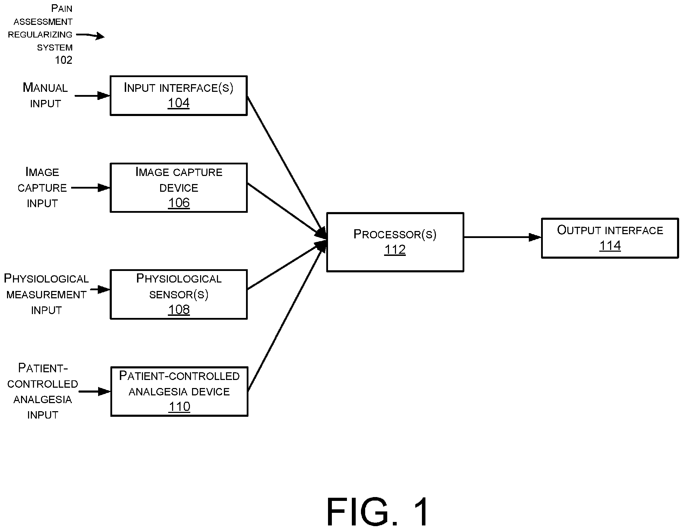

FIG. 1 illustrates a pain assessment regularizing system according to example embodiments of the present disclosure. A pain assessment regularizing system 102, according to example embodiments of the present disclosure, may include one or more sets of computer-readable instructions executable by one or more processors of a computing system to perform tasks that include collecting an input from an input source; weighing different types of collected inputs; regularizing different types of collected inputs as pain assessment factors; combining multiple heterogeneous weighted pain assessment factors; recording combined pain assessment factor in a time series; and displaying a regularized pain assessment factors trend.

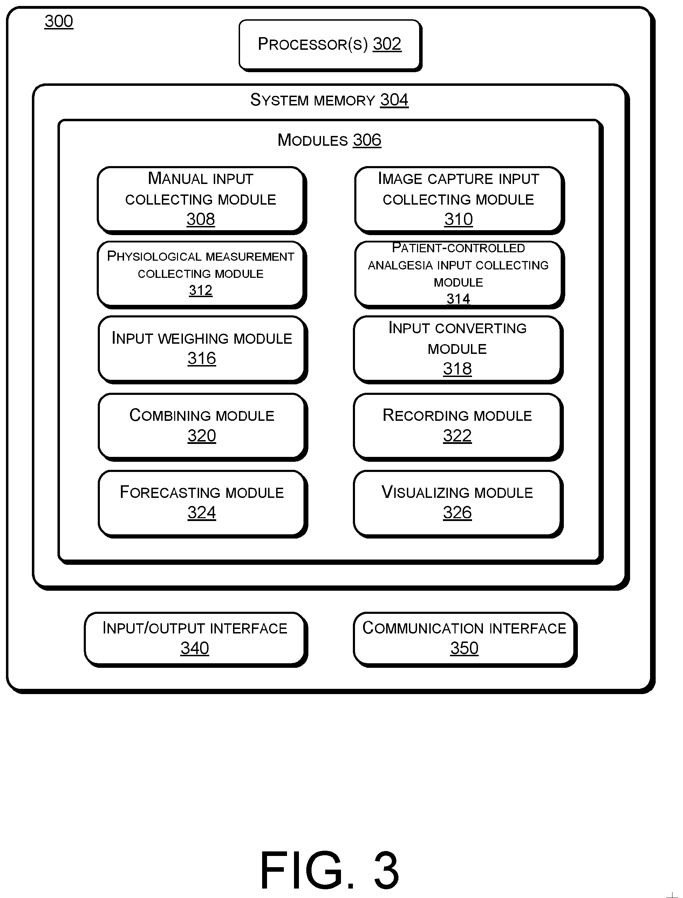

A pain assessment regularizing system 102 may be a computing system as shall be described subsequently with reference to FIG. 3. Without reiterating the subsequent description, it should be understood, for the purpose of understanding example embodiments of the present disclosure, that the pain assessment regularizing system 102 may include various input interfaces and input devices, including one or more input interface(s) 104; optionally, an image capture device 106; one or more physiological sensor(s) 108; and, optionally, a patient-controlled analgesia (PCA) device 110. Each of the various input interfaces and input device may be in communication with one or more processor(s) 112 of the pain assessment regularizing system 102 over a communication bus. The pain assessment regularizing system 102 may further include an output interface 114.

The one or more processor(s) 112 may be configured to process input from each of the input interfaces and input devices, including text input and form selection input from one or more input interface(s) 104; optionally, image capture and/or video capture input from an image capture device 106; physiological measurement input from one or more physiological sensor(s) 108; optionally, patient-controlled analgesia input from a PCA control apparatus of a PCA device 110; and the like.

Collectively, the input interfaces and input devices may cause the pain assessment regularizing system 102 to obtain inputs of multiple heterogeneous types. While some such heterogeneous types of input may be quantified measures in format, others may not be. By way of example for understanding "heterogeneous" as defined herein, inputs of each heterogeneous type of input may originate from one or more sources, and inputs of different heterogeneous types of input may originate from heterogeneous sources. For example, different, but non-heterogeneous types of input, such as text input and form selection input, may originate from non-heterogeneous sources, such as keyboards, mice, and touchscreens; heterogeneous types of input, such as video capture input, physiological measurement input, and patient-controlled analgesia input may originate from heterogeneous sources, such as an image capture device 106, a physiological sensor 108, and a PCA device 110.

Furthermore, the input interfaces and input devices may cause the pain assessment regularizing system 102 to collect information suitable to make up an electronic medical record ("EMR"), such as patient demographics, height, weight, and the like. Methods of electronic data entry and recordkeeping are known to persons skilled in the art and need not be described in detail herein for understanding example embodiments of the present disclosure.

According to example embodiments of the present disclosure, the pain assessment regularizing system 102 collects multiple inputs of heterogeneous types, each type of input representing a different source of information which may indicate a patient's actual pain intensity. A clinician may operate the pain assessment regularizing system 102 in order to regularize pain assessment factors during a single pain assessment session. Additionally, a clinician may operate the pain assessment regularizing system 102 in order to track pain assessment sessions over the course of a patient care program. Over the course of different pain assessment sessions for a same patient, the clinician may operate the pain assessment regularizing system 102 to collect multiple inputs of heterogeneous types. During each individual pain assessment session, the pain assessment regularizing system 102 may be configured to combine heterogeneous regularized pain assessment factors from different inputs, to derive a combined regularized pain assessment factor, as shall be described subsequently. During different pain assessment sessions for a same patient, the pain assessment regularizing system 102 may be configured to collect inputs for a substantially consistent set of heterogeneous types of inputs. For example, in the event that the pain assessment regularizing system 102 collects an electrocardiogram ("EKG") pulse from electrodes placed on a patient's body during a first pain assessment session, the pain assessment regularizing system 102 may be configured to collect EKG pulses during most or all subsequent pain assessment sessions.

Additionally, in the event that the pain assessment regularizing system 102 collects physiological measurement input from a physiological sensor during a first pain assessment session, the pain assessment regularizing system 102 may be configured to collect physiological measurement input from a same kind of physiological sensor during subsequent pain assessment sessions. For example, in the event that the pain assessment regularizing system 102 collects an EKG pulse from electrodes placed on a patient's body during a first pain assessment session, the pain assessment regularizing system 102 may be configured to collect EKG pulses during most or all subsequent pain assessment sessions, rather than omit the collection of EKG pulses during subsequent pain assessment sessions.

Additionally, in the event that the pain assessment regularizing system 102 collects patient-controlled analgesia input from a PCA device during a treatment event, the treatment event may be during a pain assessment session or outside a pain assessment session. Such treatment events can take place a number of times between hospitalization and hospital discharge of a patient.

According to example embodiments of the present disclosure, input interfaces and input devices may collect heterogeneous types of inputs from different sources. For example, input interface(s) 104 may be operated by a clinician to cause the pain assessment regularizing system 102 to collect manual input from the clinician. Manual input from clinicians may represent clinician appraisal of patient pain intensity, which may be based on a clinician examining a patient's body and physiology, questioning a patient, observing a patient's behavior, and the like, then appraising clinical findings therefrom based on one or more pain intensity scales designed for clinical evaluation use.

For example, one or more input interface(s) 104 may include a keyboard, a mouse, a touchscreen, and the like, and may be operated by a clinician to enter text, make selections on forms displayed on a screen, or otherwise enter data.

Additionally, manual input from clinicians may represent a clinician transcription of a patient self-report of pain, which may be based on a clinician soliciting a patient to provide answers to one or more sets of questions designed to solicit information which may be converted to an appraisal based on one or more pain intensity scales designed for clinical evaluation use. Alternately and/or additionally, a patient self-report of pain may be based on a clinician soliciting a patient to self-appraise pain intensity based on one or more pain intensity scales designed for patient self-reporting. Alternately and/or additionally, a patient self-report of pain may be a comparative self-report: i.e., based on a clinician soliciting a patient to report whether pain is more intense or less intense than during a previous self-report. Moreover, a patient self-report of pain may include a location on the patient's body where the patient is experiencing or reporting pain.

Additionally, manual input from clinicians may indicate a treatment event during a pain assessment session. An indicated treatment event may correspond to a pain management treatment which a clinician has applied to a patient during the pain assessment session. An indicated treatment event may be an ongoing treatment event, which may represent the expectation that pain experienced by the patient should subside to some extent from this pain assessment session onward over the course of the patient care program (that is, including pain assessment sessions afterwards). Additionally, one treatment event may be indicated at a time, such that subsequent treatment events override previous treatment events (i.e., it is generally best medical practice that pain management treatments should not be mixed).

It should be understood that conventional pain intensity scales designed for clinical evaluation use, or designed for patient self-reporting, are known to persons skilled in the art, and example embodiments of the present disclosure place no limitation upon how a clinician may arrive at appraisals of patient pain intensity, or how a clinician may transcribe patient self-reporting of pain, except to say that such appraisals and transcriptions should be based on a numerical, descriptive, or otherwise comparative scale, such that the clinician may input a value on this scale into the input interface(s) 104. For example, a scale may rate pain intensity on a numerical scale ranging from 0 to 10, 1 to 10, 0 to 5, 1 to 5, and the like. For example, pain intensity may be described by various characteristic terms such as "sharp," "dull," "tingling," and the like. It should further be understood that clinicians most likely perform such appraisals and transcriptions based on, respectively, consistent scales over the course of pain assessment sessions for a same patient, in order to minimize confounding of the consistency of these appraisals and transcriptions.

However, it should be understood that clinician appraisals of pain intensity and patient self-reporting of pain intensity may be inconsistent with each other, and, taken on their own, may tend to confound each other. For example, patients may over-report pain, intentionally in order to exaggerate the severity of their condition, unintentionally due to being unaccustomed to experiencing their condition, and the like. Patients may also under-report pain, fail to report pain altogether due to lack of consciousness or lucidity, and the like. At the same time, clinicians may erroneously appraise intensity of patient pain due to lack of familiarity with a patient, skewed evaluation of a patient's responses due to lack of familiarity with practical manifestations of pain intensity indications or lack of familiarity with a patient's cultural background; misleading early impressions of a patient's condition, lack of patient feedback, and the like.

Furthermore, these confounding effects may be present at the start of a patient care program, due to both the clinician and the patient being unfamiliar with the patient's condition; these confounding effects may also be present at any other time during a patient care program, due to the physiological and psychological effects of treatments (such as analgesics) affecting the patient's perception and behavior. In the event that the patient perceives pain differently than normal or expresses pain differently than normal in behavior, the patient may self-report pain intensity in a confounded manner, or a clinician may assess pain intensity in a confounded manner.

Consequently, due to the challenges of performing clinical appraisals of pain intensity and of soliciting patient self-reporting of pain, the pain assessment regularizing system 102 according to example embodiments of the present disclosure collects additional, heterogeneous inputs from non-subjective sources which further indicate a patient's actual pain intensity.

For example, a clinician may operate an image capture device 106 of the pain assessment regularizing system 102 by directing the image capture device 106 upon one of several expressive locations on a patient's body. According to example embodiments of the present disclosure, an expressive location on a patient's body may be a source of visible body expression which indicates a patient's actual pain intensity, including a patient's brows, a patient's eyes, a patient's mouth, a patient's nasolabial folds, and the like. The image capture device 106 may be directed upon any one of these expressive locations, or may be directed more broadly at a concentration of multiple expressive locations (such as a patient's face as a whole). The image capture device 106 may be configured to capture still images over time; may be configured to capture a video over time; may be configured to capture a video and extract individual frames therefrom as still images; and otherwise may generally be configured to capture images and/or video reflecting changes in visible body expression over time.

According to example embodiments of the present disclosure, an image capture device 106 may be operated while situated in a fixed position relative to a resting patient or an active patient. For example, an image capture device 106 may be situated overhead above a hospital bed where a patient may be placed in rest, such that the image capture device 106 may capture whole-body images of the patient during rest. For example, an image capture device 106 may be situated overhead above an exercise mat where a patient engages in physical therapy, such that the image capture device 106 may capture whole-body images of the patient during exercises.

Furthermore, it should be understood that, in the context of providing patient care, capturing images of patients using conventional photographic sensors may result in images which allow patients to be personally identified. In the context of patient care, such captured images may constitute personal medical information, consequently becoming subject to privacy laws of many countries and jurisdictions worldwide which govern the storage of healthcare data. According to example embodiments of the present disclosure, however, the generation and storage of personal medical information is not necessary to the operation of the pain assessment regularizing system 102, and thus the pain assessment regularizing system 102 may be configured to avoid the capture of personally identifying information, such as by configuring the image capture device 106 as follows.

According to example embodiments of the present disclosure, an image capture device 106 may include any optical sensor or non-optical sensor configured to capture images, whether optically or non-optically, while excluding visible wavelengths of light. For example, an image capture device may include an infrared sensor configured to capture thermographic images. Alternatively, an image capture device may be a visible light sensor, such as a CCD or CMOS sensor, configured to capture near-infrared wavelengths and other non-visible wavelengths of light. Alternatively, an image capture device 106 may include a depth sensor, a range sensor, stereoscopic sensors, or any other sensor configured to capture depth images based on the sensor's distance from various objects, by mapping, scanning, remote sensing, or other suitable technologies. By such configurations, an image capture device 106 may capture images excluding visual information; for example, thermographic images and depth images may exclude detailed physical appearance allowing imaged patients to be identified.

Moreover, it should be understood that an image capture device 106 need not be a part of a pain assessment regularizing system 102 according to example embodiments of the present disclosure. Alternatively, an image capture device 106 may be part of a pain assessment regularizing system 102, but may not be operated by a clinician during pain assessment sessions according to example embodiment of the present disclosure. For example, some patients, while subject to visual observation (including observation by image capture devices such as cameras), may behave in self-conscious ways, such as suppressing visible body expression, or exaggerating visible body expression. Therefore, operation of the image capture device 106 may be omitted from the operation of the pain assessment regularizing system 102 without substantially adversely impacting the derivation of regularized pain assessment factors as subsequently described. Moreover, in the event that the omission or non-operation of the image capture device 106 avoids capturing suppressed visible body expressions and avoids capturing exaggerated visible body expressions, the omission or non-operation of the image capture device 106 may even avert adverse impacts upon the derivation of regularized pain assessment factors.

Additionally, a clinician may operate one or more physiological sensor(s) 108 of the pain assessment regularizing system 102 by affixing one or more physiological sensor(s) 108 to an operative position on a patient's body. According to example embodiments of the present disclosure, a physiological sensor 108 may be any of a variety of electronic sensors operative to measure physiological phenomena, such as electrocardiography (ECG) sensors, electromyography (EMG) sensors, electroencephalography (EEG) sensors, and any other suitable electrophysiological sensors; pulse sensors or blood volume pulse (BVP) sensors utilizing any suitable underlying technology, such as transmissive pulse oximetry sensors, reflectance pulse oximetry sensors, and the like (which are also commonly known as photoplethysmography (PPG) sensors); blood pressure sensors utilizing any suitable underlying technology (including the same technology as pulse sensors as described above); respiratory rate sensors utilizing any suitable underlying technology (including the same technology as pulse sensors as described above, and further including girth sensors which detect physical chest abdominal expansion and contraction); blood carbon dioxide sensors utilizing any suitable underlying technology (including the same technology as pulse sensors as described above); galvanic skin response sensors; and the like. Operative positions for electrophysiological sensors may be at a variety of locations on a patient's body, and, with respect to EEG sensors, such operative positions can further include inside an ear canal of a patient; operative positions for pulse oximetry sensors may be over a finger of a patient, across the forehead of a patient, and the like; and similarly, operative positions for galvanic skin response sensors may be over a finger of a patient, and the like. Operative positions for girth sensors may be fitted over and across a chest and/or an abdomen of a patient, so that the girth sensor can physically expand and contract with respiration of the patient. Each such sensor may be configured in a form suitable for affixation to an operative position on a patient's body, such as an electrode, a finger clamp, a forehead band, a wristband, and the like, in accordance with relevant operative positions.

According to example embodiments of the present disclosure, multiple physiological sensors 108 which are collectively operable at substantially the same operative position on a patient's body may be encompassed in one housing, and collectively affixed to one operative position. Multiple physiological sensors 108 can, furthermore, be encoded in parallel by a multichannel encoder which is in communication with one or more processor(s) 112, so that all of the physiological sensors 108 can be active at the same time and in communication with one or more processor(s) 112 in parallel.

According to example embodiments of the present disclosure, a physiological sensor 108 may be operated while affixed to the body of a resting patient or an active patient. For example, a physiological sensor 108 may be affixed to a patient at rest in a hospital bed. For example, a physiological sensor 108 may be affixed to a patient engaging in physical therapy exercises.

It should be understood that, according to example embodiments of the present disclosure, an expressive location and an operative position need not correspond to a location where the patient is experiencing or reporting pain (as described above). An image capture device 106 may be directed without regard to where the patient is experiencing or reporting pain. A physiological sensor 108 of any particular kind may be affixed at a substantially same operative position regardless of where the patient is experiencing or reporting pain; a finger clamp may be affixed at a finger, a forehead band may be affixed at a forehead, and so on.

Additionally, during a treatment event, a PCA device 110 can be attached intravenously to a patient and can be configured to intravenously deliver an analgesic to a patient. The PCA device 110 can further provide a PCA control apparatus, which can be configured to be operated by a patient to signal the PCA device 110 to intravenously deliver a further dose of the analgesic. It should be understood that the PCA device 110 may not be configured to deliver a further dose of the analgesic upon every operation of the control apparatus by the patient, to prevent overdosing. However, according to example embodiments of the present disclosure, each operation of the PCA control apparatus, whether successfully signaling a further dose or not, can be received by a pain assessment regularizing system 102 as a patient-controlled analgesia input, since increasing frequency of operating the PCA control apparatus tends to indicate greater pain intensity.

The pain assessment regularizing system 102, by one or more quantifying computations, may convert each input into a regularized pain assessment factor. For each heterogeneous type of input, the pain assessment regularizing system 102 may perform one or more respective heterogeneous quantifying computations to derive one or more respective heterogeneous types of regularized pain assessment factor. For example, through text input and form selection input, a clinician may input quantified measures representing pain intensity scale values; thus, the pain assessment regularizing system 102 may convert pain intensity scale values into a regularized pain assessment factor by normalizing pain intensity scale values into a common scale. Thus, a "regularized pain assessment factor" according to example embodiments of the present disclosure should be understood as an input among heterogeneous types of input being mapped to a common scale.

For example, in the event that a first pain intensity scale ranges over 1 to 5 and a second pain intensity scale ranges over 0 to 10, the pain assessment regularizing system 102 may proportionately map values on both scales to values on a normalized scale ranging over 0 to 1. Furthermore, in the event that pain intensity is described according to characteristics, the pain assessment regularizing system 102 may map different characteristic terms to different values on a normalized scale. Additionally, depending on a location on the patient's body where the patient is experiencing or reporting pain, the pain assessment regularizing system 102 may increase or decrease a mapped value on a normalized scale in accordance with whether the location is a sensitive location with many pain receptors or an insensitive location with few pain receptors.

For example, through image capture input or video capture input, an image capture device 106 may capture images and/or video reflecting changes in visible body expression over time. The pain assessment regularizing system 102 may be configured to extract some number of temporally successive images from the image capture input or video capture input. For example, given an image capture device 106 capturing some sequence of still images, the pain assessment regularizing system 102 may collect successive still images at regular or non-regular intervals of time from among the sequence of still images. For example, given an image capture device 106 capturing a sequence of video, the pain assessment regularizing system 102 may collect successive still images at regular or non-regular intervals of time from the sequence of video.

The pain assessment regularizing system 102 may be configured to run a facial feature recognition learning model, which may extract one or more facial features from one or more collected still images.

A learning model, according to example embodiments of the present disclosure, may include one or more sets of computer-readable instructions executable by one or more processors of a computing system to perform tasks that include processing collected inputs and outputting features thereof. A learning model may be, for example, a layered model such as a deep neural network, which may have a fully-connected structure, may have a feedforward structure such as a convolutional neural network ("CNN"), may have a backpropagation structure such as a recurrent neural network ("RNN"), or may have other architectures suited to the computation of particular tasks. Tasks may include, for example, classification, clustering, matching, regression, and the like.

Tasks may provide output for the performance of functions such as recognizing features of facial expressions in images and/or video. A learning model may configure a computing system to extract features from collected image input as described above. For example, a learning model may be trained to configure a computing system to extract pained expression features from collected image input. Pained expression features may include, for example, brow features, closed eye features, skin folds features, mouth features, saccadic eye movements features (over time), and the like.

With regard to facial features, it should be noted for the purpose of understanding example embodiments of the present disclosure that concepts such as "brow features," "closed eye features," "skin folds features," and "mouth features," are generally high-level descriptions of an intuition that, somewhere in input images, collections of pixels corresponding to brows, eyes, skin folds, mouths, and other such familiar parts of human faces may be extracted. Labels such as "brow features," "closed eye features," "skin folds features," or "mouth features" etc., however, are generally not understood by persons skilled in the art as suggesting or being limited to any particular rules, principles, precepts, guidelines, standards, or otherwise techniques or solutions for identifying features in facial images that correspond to eyes, noses, mouths, and the like. Instead, depending on the nature of input facial images and contexts in which input facial images were captured, any technique for corresponding image features to brows, eyes, skin folds, mouths, etc. may be deemed valid by persons skilled in the art.

Pained expression features may allow determination of behavioral characteristics such as a pained emotion in an individual person observed in collected inputs. For example, by directing the image capture device 106 upon one of several expressive locations on a patient's body, the image capture device 106 may capture pained expression features which indicate that the patient is physiologically and/or psychologically pained. The learning model may extract, from a captured image, a brow feature, brow features having various shapes which may indicate that the brow of the patient is furrowed (which may be a pained expression) or the brow of the patient is relaxed. The learning model may extract, from a captured image, an eyes feature, eyes features being in states such as open, closed (which may be a pained expression), squinted (which may be a pained expression), saccadic eye movements, and the like. The learning model may extract, from a captured image, one or more skin folds features, skin folds features having various appearances which may indicate that, for example, nasolabial folds of the patient are creased (which may be a pained expression), and the like. The learning model may extract, from a captured image, a mouth feature, mouth features having various shapes which may indicate that the mouth of the patient is clenched (which may be a pained expression), that the mouth of the patient is pinched (which may be a pained expression), that the mouth of the patient is biting a lip (which may be a pained expression), that the mouth of the patient is frowning (which may be a pained expression), that the mouth of the patient is wide open (which may be a pained expression), and the like.

Moreover, a learning model may be trained to configure a computing system to extract pained gestures from collected image input. Pained gestures may include, for example, gestures of particular body parts being in particular poses, such as a clenched pose, a guarded pose (particularly a guarded pose which is still over time), a curled or fetal pose, and such poses where body parts are generally more tense, folded, drawn inward, and/or still than neutral, at-rest poses. Pained gestures may include, for example, movements of particular body parts over time, such as saccadic eye movements.

With regard to gestures, it should be noted for the purpose of understanding example embodiments of the present disclosure that gestures may be extracted from captured images according to various techniques as known to persons skilled in the art. For example, according to hand gesture tracking techniques, a pain assessment regularizing system 102 may be configured to execute a hand gesture tracking module to identify, from a captured image, individual jointed components of a hand and their positions relative to each other, then track the movement of these jointed components over a sequence of images. For example, according to skeleton tracking techniques, a pain assessment regularizing system 102 may be configured to execute a skeleton tracking module to identify, from a captured image, individual jointed components of a body and their positions relative to each other, then track the movement of these jointed components over a sequence of images.

Pained gestures may allow determination of behavioral characteristics such as a pained gesture in an individual person observed in collected inputs. For example, by directing the image capture device 106 upon a patient's body, the image capture device 106 may capture pained gestures which indicate that the patient is physiologically and/or psychologically pained. Furthermore, hand gesture tracking techniques and skeleton tracking techniques generally do not require an image capture device 106 to capture visible wavelengths of light; thus, the captured images may exclude visual information, configuring the pain assessment regularizing system 102 to avoid the capture of personally identifying information.

According to example embodiments of the present disclosure, an image capture device 106 of the pain assessment regularizing system 102 is operative to capture facial expression images of a patient over prolonged periods of time, so that the pain assessment regularizing system 102 may extract pained expression features from the captured images. While a clinician may manually observe the patient to look for pained expressions, being under a clinician's prolonged gaze may cause a patient to change behavior (for example, a patient may feel comforted by the gaze of a clinician), confounding clinical pain assessments. Moreover, pained expressions may occur ephemerally and be readily missed through human observation; clinicians generally cannot watch a patient's face for a prolonged period of time, due to having other professional duties. Pained expressions may particularly be unpredictable in the event that a patient is unconscious during observation. Consequently, an image capture device 106 of the pain assessment regularizing system 102 enables automated observation of pained expressions of a patient, not subject to clinician omission, inattentiveness, and the like.

For example, through physiological measurement input, a physiological sensor 108 may measure vital signs of a patient over time. The pain assessment regularizing system 102 may be configured to collect some number of temporally successive vital sign measurements or other physiological measurements from any physiological sensor 108. For example, given a physiological sensor 108 measuring a vital sign or another physiological measurement on an ongoing basis, the pain assessment regularizing system 102 may collect successive vital sign measurements or other physiological measurements at regular or non-regular intervals of time from the physiological sensor 108.

According to example embodiments of the present disclosure, one or more vital signs measured by a physiological sensor 108 may include blood pressure, measured by a blood pressure sensor as described above; pulse rate, pulse rate variability, pulse transit time (PTT), pulse waveform, or pulse amplitude, measured by a pulse sensor as described above; and respiratory rate or respiration time, measured by a respiratory rate sensor as described above. One or more other physiological measurements measured by a physiological sensor 108 may include galvanic skin response, skin conductance, or number of fluctuations of skin conductance (NFSC), measured by a galvanic skin response sensors as described above; cortical activity or neural activity in general, measured by an EEG sensor as described above; muscle contraction, measured by an EMG sensor or EKG sensor as described above; and blood carbon dioxide level, measured by a blood carbon dioxide sensor as described above.

Consequently, according to example embodiments of the present disclosure, during a pain assessment session, the pain assessment regularizing system may collect at least each of the following inputs from different sources: manual input from the clinician; some number of temporally successive images from an image capture device; and some number of temporally successive physiological measurements from a physiological sensor. Additionally, during a treatment event, whether the treatment is during or outside a pain assessment session, the pain assessment regularizing system may collect a patient-controlled analgesia input from a patient-controlled analgesia device.

Additionally, as described above, the pain assessment regularizing system 102 may collect successive inputs at regular intervals of time or non-regular intervals of time. The pain assessment regularizing system 102 may be configured to vary the durations of these intervals of time. For example, the pain assessment regularizing system 102 may be configured to collect inputs more frequently during earlier pain assessment sessions and less frequently during later pain assessment sessions. In this fashion, the pain assessment regularizing system 102 may be configured to collect more data points earlier in a patient care program, to assist in forecasting a pain trend as shall be described subsequently.

Additionally, according to example embodiments of the present disclosure, the pain assessment regularizing system 102 may weigh inputs from each different source, in order to discount potentially confounding effects upon each input. The pain assessment regularizing system 102 may up-weigh some inputs; may down-weigh some inputs; and may neither up-weigh nor down-weigh some inputs.

The pain assessment regularizing system 102 may weigh an input based on a trained weight set. A learning model (for example, any learning model as described above, without limitation thereto) according to example embodiments of the present disclosure may be trained by inputting one or more sample datasets into the learning model. The training of the learning model may further be performed on a loss function, wherein the learning model extracts labeled features from the sample datasets and optimizes the loss function. Based thereon, the learning model may generate and update weight sets over some number of epochs of training, outputting a trained weight set. The pain assessment regularizing system 102 may thereafter be configured to weigh the inputs based on a weight set trained in such a fashion.

The pain assessment regularizing system 102 may weigh an input differently depending on timing of a pain assessment session. For example, during an initial pain assessment session and early pain assessment sessions, manual input has heightened likelihood of being confounded due to both the clinician and the patient being unfamiliar with the patient's condition; thus, for early pain assessment sessions, the pain assessment regularizing system 102 may down-weigh manual input representing clinician appraisal of patient pain intensity, and/or may down-weigh manual input representing a clinician transcription of a patient self-report of pain. A patient self-report may be down-weighted more than a clinician appraisal, reflecting relative trustworthiness of each.

For example, during a pain assessment session which includes an indicated treatment event (as described above) which may affect the patient's perception and/or behavior, such as pain management medication, manual input has heightened likelihood of being confounded due to a patient's sense of pain being suppressed. Thus, for pain assessment sessions following a treatment, the pain assessment regularizing system 102 may down-weigh manual input representing clinician appraisal of patient pain intensity, and/or may down-weigh manual input representing a clinician transcription of a patient self-report of pain. A patient self-report may be down-weighted more than a clinician appraisal, reflecting relative trustworthiness of each.

For example, during later pain assessment sessions, manual input has heightened likelihood of being less confounded due to both the clinician and the patient being familiar with the patient's condition; thus, for early pain assessment sessions, the pain assessment regularizing system 102 may down-weigh manual input representing clinician appraisal of patient pain intensity, and/or may down-weigh manual input representing a clinician transcription of a patient self-report of pain. A patient self-report may be down-weighted more than a clinician appraisal, reflecting relative trustworthiness of each.

For example, during a pain assessment session which follows hospitalization of the patient, or during a pain assessment session which follows hospital discharge of the patient, all inputs have heightened likelihood of being confounded due to the patient being relocated from a habitual environment to a different environment, which may induce additional stresses upon the patient which exaggerate pain. Thus, for pain assessment sessions following hospitalization or hospital discharge, the pain assessment regularizing system 102 may down-weigh all inputs from all sources. A patient self-report may be down-weighted more than other inputs, as the patient being relocated may tend to be sensitive to the change in environment, which may lead to exaggeration of self-reported pain.

Additionally, in the event that an earlier indicated treatment event is in conflict with a comparative self-report, the pain assessment regularizing system 102 may down-weigh manual input representing a clinician transcription of a patient self-report of pain. For example, an earlier indicated treatment event may generally represent an expectation that pain intensity should subside to some extent; however, a patient self-report during the same pain assessment session which indicates that pain is more intense than during a previous self-report is in contrary to such expectations. Such a conflict (assuming that the treatment is effective) may suggest that the patient self-report is confounded and therefore unreliable.

Additionally, overall, the pain assessment regularizing system 102 may be configured to up-weigh collected image input over collected manual input, may be configured to up-weigh collected physiological measurement input over collected manual input, and may be configured to up-weigh collected patient-controlled analgesia input over collected manual input. Since image input, physiological measurement input, and patient-controlled analgesia input may generally be less subjective regarding pain assessment than manual input (since manual input generally includes conclusions as to pain intensity, while image input, physiological measurement input, and patient-controlled analgesia input do not inherently include such conclusions), image input, physiological measurement input, and patient-controlled analgesia input may each be up-weighted relative to manual input.

Additionally, as described above, the pain assessment regularizing system 102 may convert each collected input into a regularized pain assessment factor. For example, as described above, the pain assessment regularizing system 102 may have converted manually input pain intensity scale values into a regularized pain assessment factor by normalizing pain intensity scale values into a common scale. The pain assessment regularizing system 102 may now also convert collected image input into a regularized pain assessment factor, may also convert collected physiological measurement input into a regularized pain assessment factor, and may also convert collected patient-controlled analgesia input into a regularized pain assessment factor. Each of the regularized pain assessment factors may be on a scale over the same numerical range. For example, in the event that the pain intensity scale values are converted to a normalized scale ranging over 0 to 1, the other regularized pain assessment factors may also be on a normalized scale ranging over 0 to 1. Thus, a "regularized pain assessment factor" according to example embodiments of the present disclosure should be understood as an input among heterogeneous types of input being mapped to a common scale.

For example, the pain assessment regularizing system 102 may convert a collected image input to a regularized pain assessment factor by extracting a brow feature from a collected image, then placing the extracted brow feature along a scale of known shapes of brow features from least intense in pain (e.g., least furrowed in shape) to most intense in pain (e.g., most furrowed in shape).

For example, the pain assessment regularizing system 102 may convert a collected image input to a regularized pain assessment factor by extracting an eyes feature from a collected image, then placing the extracted eyes feature along a scale of known shapes of eyes features from least intense in pain (e.g., least extreme in being open or being squinted or closed) to most intense in pain (e.g., most extreme in being open or being squinted or closed).

For example, the pain assessment regularizing system 102 may convert a collected image input to a regularized pain assessment factor by extracting a skin folds feature from a collected image, then placing the extracted skin folds feature along a scale of known shapes of skin folds features from least intense in pain (e.g., least creased in shape) to most intense in pain (e.g., most creased in shape).

For example, the pain assessment regularizing system 102 may convert a collected image input to a regularized pain assessment factor by extracting a mouth feature from a collected image, then placing the extracted mouth feature along a scale of known shapes of mouth features from least intense in pain (e.g., most relaxed in shape) to most intense in pain (e.g., most stressed in shape, such as being clenched, being pinched, biting a lip, frowning, being wide open, and the like).

For example, the pain assessment regularizing system 102 may convert a collected physiological measurement input to a regularized pain assessment factor by placing measured blood pressure along a scale of blood pressure values from least intense in pain (e.g., close to 120/80 in systolic/diastolic blood pressure in mmHg) to most intense in pain (e.g., being high above 120/80 in systolic/diastolic blood pressure in mmHg).

For example, the pain assessment regularizing system 102 may convert a collected physiological measurement input to a regularized pain assessment factor by placing measured pulse rate or pulse waveform along a scale of pulse rate values or pulse waveform patterns from least intense in pain (e.g., close to a normal resting bpm measurement for the patient's demographics, height, weight, and the like according to an EMR) to most intense in pain (e.g., high above a normal resting bpm measurement for the patient's demographics, height, weight, and the like according to an EMR).

For example, the pain assessment regularizing system 102 may convert a collected physiological measurement input to a regularized pain assessment factor by placing measured respiratory rate along a scale of respiratory rate values from least intense in pain (e.g., close to normal breaths per minute at rest for the patient's demographics, height, weight, and the like according to an EMR) to most intense in pain (e.g., high above normal breaths per minute at rest for the patient's demographics, height, weight, and the like according to an EMR).

For example, the pain assessment regularizing system 102 may convert a collected physiological measurement input to a regularized pain assessment factor by placing measured galvanic skin response along a scale of galvanic skin response values from least intense in pain (e.g., high electrical resistance) to most intense in pain (e.g., low electrical resistance arising from heightened sweat production).

For example, the pain assessment regularizing system 102 may convert a collected physiological measurement input to a regularized pain assessment factor by placing measured cortical activity along a scale of cortical activity values from least intense in pain (e.g., close to a normal cortical activity level at rest for the patient's demographics, height, weight, and the like according to an EMR) to most intense in pain (e.g., high above normal cortical activity level at rest for the patient's demographics, height, weight, and the like according to an EMR).

For example, the pain assessment regularizing system 102 may convert a collected physiological measurement input to a regularized pain assessment factor by placing measured muscle contraction along a scale of muscle contraction measurements from least intense in pain (e.g., lack of muscle contraction) to most intense in pain (e.g., intense muscle contraction, such as at the forehead, sides of the head, abdomen indicating a fetal or guarded position, and the like; saccadic eye movements; pained expressions; or quivering muscle contraction).

For example, the pain assessment regularizing system 102 may convert a collected physiological measurement input to a regularized pain assessment factor by placing measured blood carbon dioxide level along a scale of blood carbon dioxide values from least intense in pain (e.g., close to a normal carbon dioxide level at rest for the patient's demographics, height, weight, and the like according to an EMR) to most intense in pain (e.g., high above normal carbon dioxide level at rest for the patient's demographics, height, weight, and the like according to an EMR).

For example, the pain assessment regularizing system 102 may convert a collected patient-controlled analgesia input to a regularized pain assessment factor by placing frequency of operations of a PCA control apparatus along a scale of frequencies from least intense in pain (i.e., least frequent operations of the PCA control apparatus) to most intense in pain (i.e., most frequent operations of the PCA control apparatus).

The pain assessment regularizing system 102 may combine heterogeneous regularized pain assessment factors from different inputs, to derive a combined regularized pain assessment factor. A set of regularized pain assessment factors may be combined in the event that they are concurrent - that is, if they are based on substantially concurrently collected inputs. For example, for image inputs and physiological measurement inputs, which the pain assessment regularizing system 102 may successively collect at regular or non-regular intervals of time, the inputs may be concurrent with each other if they were collected substantially at the same time. However, for manual inputs, which the pain assessment regularizing system 102 may only collect once for a pain assessment session, the manual inputs may be concurrent with other inputs if they were collected during a same pain assessment session. For each combined regularized pain assessment factor, a timestamp may be set in accordance with the time of collection of the image inputs and/or the physiological measurement inputs, these times being more granular than the time of collection of the manual inputs.

For example, the pain assessment regularizing system 102 may be configured to compute a sum of a set of concurrent regularized pain assessment factors. For example, the pain assessment regularizing system 102 may be configured to compute a weighted sum of a set of concurrent regularized pain assessment factors (where some of the factors may be up-weighted, some of the factors may be down-weighted, and some of the factors may be neither up-weighted nor down-weighted). For example, the pain assessment regularizing system 102 may be configured to compute a mean of a set of concurrent pain assessment factors. For example, the pain assessment regularizing system may be configured to compute a weighted mean of a set of concurrent pain assessment factors (where some of the factors may be up-weighted, some of the factors may be down-weighted, and some of the factors may be neither up-weighted nor down-weighted).

The pain assessment regularizing system 102 may derive successive combined regularized pain assessment factors from successive sets of collected inputs at regular intervals of time or non-regular intervals of time. The pain assessment regularizing system 102 may further organize these successive combined regularized pain assessment factors in time order by recording each combined regularized pain assessment factor in a time series. A time series may be a series of data records indexed by time, and the pain assessment regularizing system 102 may be configured to write a combined regularized pain assessment factor into each record of a time series, the record being indexed by time of collection.

The pain assessment regularizing system 102 may furthermore forecast a trend of the time series of combined regularized pain assessment factors. The pain assessment regularizing system 102 may forecast a trend by fitting a regression model to the time series. A regression model, according to example embodiments of the present disclosure, may be fitted to values of at least one independent variable (i.e., time of the time series) and at least one dependent variable (i.e., the value of each combined regularized pain assessment factor at a time) by computing parameters of one or more functions. A regression model may be fitted by computing parameters of a function based on observing some number of measurements of values of each of the variables (i.e., the values recorded in each record of the time series), and the parameterized function may then be operative to receive a value of the independent variable as input (i.e., future times beyond a latest record in the time series) and output a value of the dependent variable.

In the course of fitting the regression model, the pain assessment regularizing system 102 may perform regression computations such as least squares regression, linear regression, and the like as known to persons skilled in the art.

Thus, by the above-described fitting of a regression model, the pain assessment regularizing system 102 may forecast a regularized pain assessment trend. The pain assessment regularizing system 102 may further visualize the regularized pain assessment trend. For example, the pain assessment regularizing system 102 may be configured to display a visualization of the time series on an output interface 114 (such as a display screen) of the pain assessment regularizing system 102.

For example, a visualization may be a line graph, a bar graph, a bubble graph, or otherwise any suitable visualization which displays at least a time dimension of the time series and a regularized pain assessment factor dimension of the time series. Each record of the time series may be placed on the visualization based on the time dimension and the regularized pain assessment factor dimension.

Moreover, the pain assessment regularizing system 102 may, for future times beyond a latest record in the time series, input future times into the fitted regression model and obtain corresponding outputs. These pairs of inputs and outputs may further be placed on the visualization in order to visualize the forecast regularized pain assessment trend alongside visualizing the time series.

Thus, according to example embodiments of the present disclosure, a clinician may operate the pain assessment regularizing system 102 to autonomously observe pained expressions, physiological measurements, and patient-controlled analgesia input of a patient, in order to systematically collect data inputs which may be converted to pain assessment factors, enabling much more pain intensity indicating data to be collected, at predictable intervals, than a clinician is able to collect manually, and enabling data to be collected in a manner sensitive to the progression of a patient care program. The pain assessment regularizing system 102, by collecting this data, may combine it with clinical appraisals of pain intensity and patient self-reporting of pain intensity, weighing each factor appropriately in a manner sensitive to the progression of a patient care program, so as to lessen confounding effects of subjective pain assessment, without outright discounting the professional insights that clinicians may provide or the personal perspective that patients may self-report. The pain assessment regularizing system 102 may generate a time series of regularized pain assessment factors, and further forecast a regularized pain assessment trend. A clinician may further operate the pain assessment regularizing system 102 to review a visualization of both the time series and the forecast, providing the clinician with rigorously sampled and analytically predicted data which cannot be derived through manual and mental efforts, which may improve the efficacy of the clinician's pain management upon the patient during the course of the patient care program. By reviewing forecast pain assessment trends, the clinician may better avoid over-managing pain (such as due to over-prescribing medication), which may numb the patient's response to treatment and lead to outcomes such as lethargy (by biological activity of analgesics, for example) and lowered physical activity, heightened risk of respiratory system depression, chemical or psychological dependency on pain medication, and the like; may better avoid under-managing pain (such as due to under-prescribing medication), which may cause undue distress to the patient; and may manage pain to achieve more precise outcomes, such as prescribing and timing medication to effectively relieve pain on a rapid-acting basis immediately preceding physical therapy (wherein the degree of precision and personalization required may not be easily achieved based on subjective human judgment).

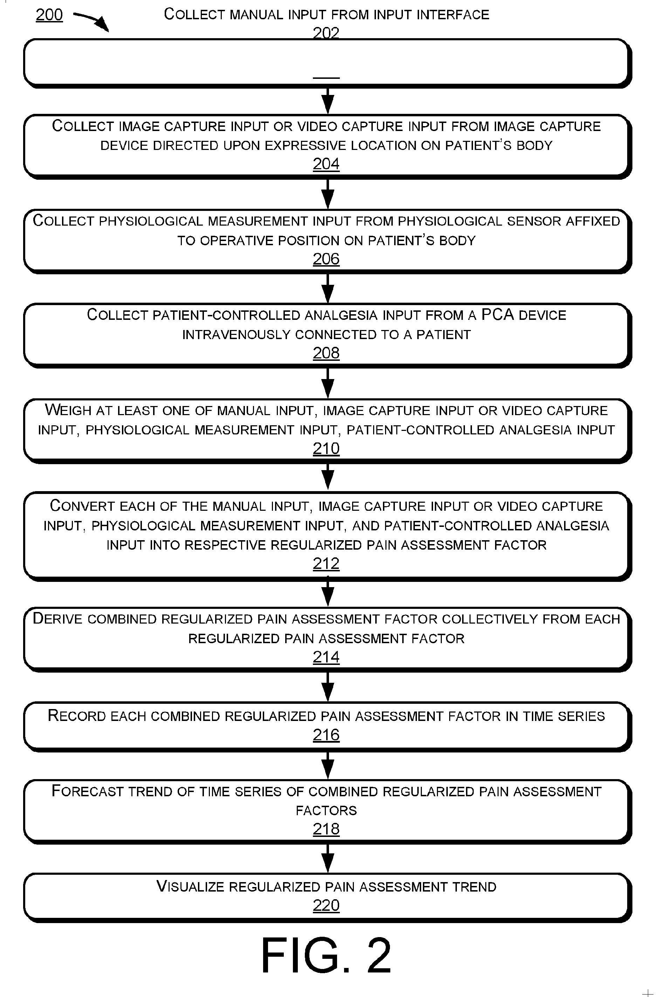

FIG. 2 illustrates a flowchart of a pain assessment factor regularizing method 200 according to example embodiments of the present disclosure.

At a step 202, a pain assessment regularizing system collects manual input from an input interface of the pain assessment regularizing system.

As described above, one or more input interface(s) of a pain assessment regularizing system may include a keyboard, a mouse, a touchscreen, and the like, and may be operated by a clinician to enter text, make selections on forms displayed on a screen, or otherwise enter data. It should be understood that, according to example embodiments of the present disclosure, a clinician may also provide the input interface to a patient and instruct the patient to enter text, make selections on forms displayed on a screen, or otherwise enter data under the clinician's supervision.

For example, input interface(s) may be operated by a clinician to cause the pain assessment regularizing system to collect manual input from the clinician. Manual input from clinicians may represent clinician appraisal of patient pain intensity, which may be based on a clinician examining a patient's body and physiology, questioning a patient, observing a patient's behavior, and the like, then appraising clinical findings therefrom based on one or more pain intensity scales designed for clinical evaluation use.

Additionally, manual input from clinicians may represent a clinician (or a patient under clinician supervision) transcription of a patient self-report of pain, which may be based on a clinician soliciting a patient to provide answers to one or more sets of questions designed to solicit information which may be converted to an appraisal based on one or more pain intensity scales designed for clinical evaluation use. Alternately and/or additionally, a patient self-report of pain may be based on a clinician soliciting a patient to self-appraise pain intensity based on one or more pain intensity scales designed for patient self-reporting.

According to example embodiments of the present disclosure, it should be understood that step 202 and steps 204 and 206 below may be performed during a same pain assessment session wherein a patient is under clinician observation and care. During such a session, while step 202 may be performed one time, steps 204 and 206 may be performed any number of times; moreover, these steps may be performed in any order relative to each other, or concurrently or non-concurrently relative to each other, without limitation.

At a step 204, the pain assessment regularizing system collects image capture input or video capture input from an image capture device of the pain assessment regularizing system directed upon an expressive location on a patient's body.

According to example embodiments of the present disclosure, an expressive location on a patient's body may be a source of visible body expression which indicates a patient's actual pain intensity, including a patient's brows, a patient's eyes, a patient's mouth, a patient's nasolabial folds, and the like. The image capture device may be directed upon any one of these expressive locations, or may be directed more broadly at a concentration of multiple expressive locations

(such as a patient's face as a whole). The image capture device may be configured to capture still images over time; may be configured to capture a video over time; may be configured to capture a video and extract individual frames therefrom as still images; and otherwise may generally be configured to capture images and/or video reflecting changes in visible body expression over time.

At a step 206, the pain assessment regularizing system collects physiological measurement input from a physiological sensor of the pain assessment regularizing system affixed to an operative position on a patient's body.

According to example embodiments of the present disclosure, a physiological sensor may be any of a variety of electronic sensors operative to measure physiological phenomena, such as ECG sensors, EMG sensors, EEG sensors and any other suitable electrophysiological sensors; pulse sensors or BVP sensors utilizing any suitable underlying technology, such as transmissive pulse oximetry sensors, reflectance pulse oximetry sensors, and the like (i.e., PPG sensors); blood pressure sensors utilizing any suitable underlying technology (including the same technology as pulse sensors as described above); respiratory rate sensors utilizing any suitable underlying technology (including the same technology as pulse sensors as described above, and further including girth sensors which detect physical chest abdominal expansion and contraction); blood carbon dioxide sensors utilizing any suitable underlying technology (including the same technology as pulse sensors as described above); galvanic skin response sensors; and the like. Operative positions for electrophysiological sensors may be at a variety of locations on a patient's body, and, with respect to EEG sensors, such operative positions can further include inside an ear canal of a patient; operative positions for pulse oximetry sensors may be over a finger of a patient, across the forehead of a patient, and the like; and similarly, operative positions for galvanic skin response sensors may be over a finger of a patient and the like. Operative positions for girth sensors may be fitted over and across a chest and/or an abdomen of a patient, so that the girth sensor can physically expand and contract with respiration of the patient. Each such sensor may be configured in a form suitable for affixation to an operative position on a patient's body, such as an electrode, a finger clamp, a forehead band, a wristband, and the like, in accordance with relevant operative positions.

It should be understood that, according to example embodiments of the present disclosure, an expressive location and an operative position need not correspond to a location where the patient is experiencing or reporting pain. An image capture device may be directed without regard to where the patient is experiencing or reporting pain. A physiological sensor of any particular kind may be affixed at a substantially same operative position regardless of where the patient is experiencing or reporting pain; a finger clamp may be affixed at a finger, a forehead band may be affixed at a forehead, and so on.

At a step 208, the pain assessment regularizing system collects patient-controlled analgesia input from a PCA device intravenously connected to a patient.

It should be understood that the location of a PCA device is irrelevant as long as it is intravenously connected to the patient, and the PCA control apparatus is accessible by the patient.

It should be understood that step 204, step 206, and step 208 as described above may each include the pain assessment regularizing system collecting successive inputs at regular intervals of time or non-regular intervals of time. The pain assessment regularizing system may further be configured to vary the durations of these intervals of time. For example, the pain assessment regularizing system may be configured to collect inputs more frequently during earlier pain assessment sessions and less frequently during later pain assessment sessions. In this fashion, the pain assessment regularizing system may be configured to collect more data points earlier in a patient care program, to assist in forecasting a pain trend as shall be described subsequently.

At a step 210, the pain assessment regularizing system weighs at least one of the manual input, the image capture input or video capture input, and the physiological measurement input.

In order to discount potentially confounding effects upon each input, the pain assessment regularizing system may up-weigh some inputs; may down-weigh some inputs; and may neither up-weigh nor down-weigh some inputs. The pain assessment regularizing system may weigh an input differently depending on timing of a pain assessment session. For example, during an initial pain assessment session and early pain assessment sessions, manual input has heightened likelihood of being confounded due to both the clinician and the patient being unfamiliar with the patient's condition; thus, for early pain assessment sessions, the pain assessment regularizing system may down-weigh manual input representing clinician appraisal of patient pain intensity, and/or may down-weigh manual input representing a clinician transcription of a patient self-report of pain. A patient self-report may be down-weighted more than a clinician appraisal, reflecting relative trustworthiness of each.

Additionally, overall, the pain assessment regularizing system may be configured to up-weigh collected image input over collected manual input, may be configured to up-weigh collected physiological measurement input over collected manual input, and may be configured to up-weigh collected patient-controlled analgesia input over collected manual input. Since image input, physiological measurement input, and patient-controlled analgesia input may generally be less subjective regarding pain assessment than manual input (since manual input generally includes conclusions as to pain intensity, while image input, physiological measurement input, and patient-controlled analgesia input do not inherently include such conclusions), image input, physiological measurement input, and patient-controlled analgesia input may each be up-weighted relative to manual input.

At a step 212, the pain assessment regularizing system converts each of the manual input, the image capture input or video capture input, the physiological measurement input, and the patient-controlled analgesia input into a respective regularized pain assessment factor.

For example, the pain assessment regularizing system, by one or more quantifying computations, may convert each input into a regularized pain assessment factor. For each heterogeneous type of input, the pain assessment regularizing system may perform one or more respective heterogeneous quantifying computations to derive one or more respective heterogeneous types of regularized pain assessment factor. For example, through text input and form selection input, a clinician may input quantified measures representing pain intensity scale values; thus, the pain assessment regularizing system may convert pain intensity scale values into a regularized pain assessment factor by normalizing pain intensity scale values into a common scale. For example, in the event that a first pain intensity scale ranges over 1 to 5 and a second pain intensity scale ranges over 0 to 10, the pain assessment regularizing system may proportionately map values on both scales to values on a normalized scale ranging over 0 to 1.

The pain assessment regularizing system may also convert collected image input into a regularized pain assessment factor, may also convert collected physiological measurement input into a regularized pain assessment factor, and may also convert collected patient-controlled analgesia input into a regularized pain assessment factor. Each of the regularized pain assessment factors may be on a scale over the same numerical range. For example, in the event that the pain intensity scale values are converted to a normalized scale ranging over 0 to 1, the other regularized pain assessment factors may also be on a normalized scale ranging over 0 to 1.

For example, the pain assessment regularizing system may convert a collected image input to a regularized pain assessment factor by extracting a brow feature from a collected image, then placing the extracted brow feature along a scale of known shapes of brow features from least intense in pain (e.g., least furrowed in shape) to most intense in pain (e.g., most furrowed in shape).

For example, the pain assessment regularizing system may convert a collected image input to a regularized pain assessment factor by extracting an eyes feature from a collected image, then placing the extracted eyes feature along a scale of known shapes of eyes features from least intense in pain (e.g., least extreme in being open or being squinted or closed) to most intense in pain (e.g., most extreme in being open or being squinted or closed).

For example, the pain assessment regularizing system may convert a collected image input to a regularized pain assessment factor by extracting a skin folds feature from a collected image, then placing the extracted skin folds feature along a scale of known shapes of skin folds features from least intense in pain (e.g., least creased in shape) to most intense in pain (e.g., most creased in shape).

For example, the pain assessment regularizing system may convert a collected image input to a regularized pain assessment factor by extracting a mouth feature from a collected image, then placing the extracted mouth feature along a scale of known shapes of mouth features from least intense in pain (e.g., most relaxed in shape) to most intense in pain (e.g., most stressed in shape, such as being clenched, being pinched, biting a lip, frowning, being wide open, and the like).

For example, the pain assessment regularizing system may convert a collected physiological measurement input to a regularized pain assessment factor by placing measured blood pressure along a scale of blood pressure values from least intense in pain (e.g., close to 120/80 in systolic/diastolic blood pressure in mmHg) to most intense in pain (e.g., being high above 120/80 in systolic/diastolic blood pressure in mmHg).

For example, the pain assessment regularizing system may convert a collected physiological measurement input to a regularized pain assessment factor by placing measured pulse rate or pulse waveform along a scale of pulse rate values or pulse waveform patterns from least intense in pain (e.g., close to a normal resting bpm measurement for the patient's demographics, height, weight, and the like according to an EMR) to most intense in pain (e.g., high above a normal resting bpm measurement for the patient's demographics, height, weight, and the like according to an EMR).

For example, the pain assessment regularizing system may convert a collected physiological measurement input to a regularized pain assessment factor by placing measured respiratory rate along a scale of respiratory rate values from least intense in pain (e.g., close to normal breaths per minute at rest for the patient's demographics, height, weight, and the like according to an EMR) to most intense in pain (e.g., high above normal breaths per minute at rest for the patient's demographics, height, weight, and the like according to an EMR).

For example, the pain assessment regularizing system may convert a collected physiological measurement input to a regularized pain assessment factor by placing measured galvanic skin response along a scale of galvanic skin response values from least intense in pain (e.g., high electrical resistance) to most intense in pain (e.g., low electrical resistance arising from heightened sweat production).

For example, the pain assessment regularizing system may convert a collected physiological measurement input to a regularized pain assessment factor by placing measured cortical activity along a scale of cortical activity values from least intense in pain (e.g., close to a normal cortical activity level at rest for the patient's demographics, height, weight, and the like according to an EMR) to most intense in pain (e.g., high above normal cortical activity level at rest for the patient's demographics, height, weight, and the like according to an EMR).

For example, the pain assessment regularizing system may convert a collected physiological measurement input to a regularized pain assessment factor by placing measured muscle contraction along a scale of muscle contraction measurements from least intense in pain (e.g., lack of muscle contraction) to most intense in pain (e.g., intense muscle contraction, such as at the forehead, sides of the head, abdomen indicating a fetal or guarded position, and the like; saccadic eye movements; pained expressions; or quivering muscle contraction).

For example, the pain assessment regularizing system may convert a collected physiological measurement input to a regularized pain assessment factor by placing measured blood carbon dioxide level along a scale of blood carbon dioxide values from least intense in pain (e.g., close to a normal carbon dioxide level at rest for the patient's demographics, height, weight, and the like according to an EMR) to most intense in pain (e.g., high above normal carbon dioxide level at rest for the patient's demographics, height, weight, and the like according to an EMR).

For example, the pain assessment regularizing system may convert a collected patient-controlled analgesia input to a regularized pain assessment factor by placing frequency of operations of a PCA control apparatus along a scale of frequencies from least intense in pain (i.e., least frequent operations of the PCA control apparatus) to most intense in pain (i.e., most frequent operations of the PCA control apparatus).

At a step 214, the pain assessment regularizing system derives a combined regularized pain assessment factor collectively from each regularized pain assessment factor.

A set of regularized pain assessment factors may be combined in the event that they are concurrent - that is, if they are based on substantially concurrently collected inputs. For example, for image inputs and physiological measurement inputs, which the pain assessment regularizing system may successively collect at regular or non-regular intervals of time, the inputs may be concurrent with each other if they were collected substantially at the same time. However, for manual inputs, which the pain assessment regularizing system may only collect once for a pain assessment session, the manual inputs may be concurrent with other inputs if they were collected during a same pain assessment session. For each combined regularized pain assessment factor, a timestamp may be set in accordance with the time of collection of the image inputs and/or the physiological measurement inputs, these times being more granular than the time of collection of the manual inputs.

For example, the pain assessment regularizing system may be configured to compute a sum of a set of concurrent regularized pain assessment factors. For example, the pain assessment regularizing system may be configured to compute a weighted sum of a set of concurrent regularized pain assessment factors (where some of the factors may be up-weighted, some of the factors may be down-weighted, and some of the factors may be neither up-weighted nor down-weighted). For example, the pain assessment regularizing system may be configured to compute a mean of a set of concurrent pain assessment factors. For example, the pain assessment regularizing system may be configured to compute a weighted mean of a set of concurrent pain assessment factors (where some of the factors may be up-weighted, some of the factors may be down-weighted, and some of the factors may be neither up-weighted nor down-weighted).

At a step 216, the pain assessment regularizing system records each combined regularized pain assessment factor in a time series.

The pain assessment regularizing system may derive successive combined regularized pain assessment factors from successive sets of collected inputs at regular intervals of time or non-regular intervals of time. The pain assessment regularizing system may further organize these successive combined regularized pain assessment factors in time order by. A time series may be a series of data records indexed by time, and the pain assessment regularizing system may be configured to write a combined regularized pain assessment factor into each record of a time series, the record being indexed by time of collection.

At a step 218, the pain assessment regularizing system forecasts a trend of the time series of combined regularized pain assessment factors.

The pain assessment regularizing system may forecast a trend by fitting a regression model to the time series. A regression model, according to example embodiments of the present disclosure, may be fitted to values of at least one independent variable (i.e., time of the time series) and at least one dependent variable (i.e., the value of each combined regularized pain assessment factor at a time) by computing parameters of one or more functions. A regression model may be fitted by computing parameters of a function based on observing some number of measurements of values of each of the variables (i.e., the values recorded in each record of the time series), and the parameterized function may then be operative to receive a value of the independent variable as input (i.e., future times beyond a latest record in the time series) and output a value of the dependent variable.

In the course of fitting the regression model, the pain assessment regularizing system may perform regression computations such as least squares regression, linear regression, and the like as known to persons skilled in the art.

At a step 220, the pain assessment regularizing system visualizes the regularized pain assessment trend.

For example, the pain assessment regularizing system may be configured to display a visualization of the time series on an output interface (such as a display screen) of the pain assessment regularizing system.

For example, a visualization may be a line graph, a bar graph, a bubble graph, or otherwise any suitable visualization which displays at least a time dimension of the time series and a regularized pain assessment factor dimension of the time series. Each record of the time series may be placed on the visualization based on the time dimension and the regularized pain assessment factor dimension.

Moreover, the pain assessment regularizing system may, for future times beyond a latest record in the time series, input future times into the fitted regression model and obtain corresponding outputs. These pairs of inputs and outputs may further be placed on the visualization in order to visualize the forecast regularized pain assessment trend alongside visualizing the time series.

FIG. 3 illustrates an example system 300 for implementing the processes and methods described above for implementing regularizing pain assessment factors.

The techniques and mechanisms described herein may be implemented by multiple instances of the system 300, as well as by any other computing device, system, and/or environment. The system 300 shown in FIG. 3 is only one example of a system and is not intended to suggest any limitation as to the scope of use or functionality of any computing device utilized to perform the processes and/or procedures described above. Other well-known computing devices, systems, environments and/or configurations that may be suitable for use with the embodiments include, but are not limited to, personal computers, server computers, hand-held or laptop devices, multiprocessor systems, microprocessor-based systems, set top boxes, game consoles, programmable consumer electronics, network PCs, minicomputers, mainframe computers, distributed computing environments that include any of the above systems or devices, implementations using field programmable gate arrays ("FPGAs") and application specific integrated circuits ("ASICs"), and/or the like.

The system 300 may include one or more processors 302 and system memory 304 communicatively coupled to the processor(s) 302. The processor(s) 302 and system memory 304 may be physical or may be virtualized and/or distributed. The processor(s) 302 may execute one or more modules and/or processes to cause the processor(s) 302 to perform a variety of functions. In embodiments, the processor(s) 302 may include a central processing unit ("CPU"), a graphics processing unit ("GPU"), both CPU and GPU, or other processing units or components known in the art. Additionally, each of the processor(s) 302 may possess its own local memory, which also may store program modules, program data, and/or one or more operating systems.

Depending on the exact configuration and type of the system 300, the system memory 304 may be volatile, such as RAM, non-volatile, such as ROM, flash memory, miniature hard drive, memory card, and the like, or some combination thereof. The system memory 304 may include one or more computer-executable modules 906 that are executable by the processor(s) 302.

The modules 306 may include, but are not limited to, a manual input collecting module 308, an image capture input collecting module 310, a physiological measurement collecting module 312, a patient-controlled analgesia input collecting module 314, an input weighing module 316, an input converting module 318, a combining module 320, a recording module 322, a forecasting module 324, and a visualizing module 326.

The manual input collecting module 308 is configured to collect manual input from an input interface of the pain assessment regularizing system as described above with reference to FIG. 2.

The image capture input collecting module 310 may be configured to collect image capture input or video capture input from an image capture device of the pain assessment regularizing system directed upon an expressive location on a patient's body as described above with reference to FIG. 2.

The physiological measurement collecting module 312 is configured to collect physiological measurement input from a physiological sensor of the pain assessment regularizing system affixed to an operative position on a patient's body as described above with reference to FIG. 2.

The patient-controlled analgesia input collecting module 314 may be configured to collects patient-controlled analgesia input from a PCA device intravenously connected to a patient as described above with reference to FIG. 2.

The input weighing module 316 is configured to weigh at least one of the manual input, the image capture input or video capture input, and the physiological measurement input as described above with reference to FIG. 2.

The input converting module 318 is configured to convert each of the manual input, the image capture input or video capture input, and the physiological measurement input into a respective regularized pain assessment factor as described above with reference to FIG. 2.

The combining module 320 is configured to derive a combined regularized pain assessment factor collectively from each regularized pain assessment factor as described above with reference to FIG. 2.

The recording module 322 is configured to record each combined regularized pain assessment factor in a time series as described above with reference to FIG. 2.

The forecasting module 324 may be configured to forecast a trend of the time series of combined regularized pain assessment factors as described above with reference to FIG. 2.

The visualizing module 326 is configured to visualize the regularized pain assessment trend as described above with reference to FIG. 2.

The system 300 may additionally include an input/output (I/O) interface 340 and a communication module 350 allowing the system 300 to communicate with other systems and devices over a network. The network may include the Internet, wired media such as a wired network or direct-wired connections, and wireless media such as acoustic, radio frequency ("RF"), infrared, and other wireless media.

Some or all operations of the methods described above can be performed by execution of computer-readable instructions stored on a computer-readable storage medium, as defined below. The term "computer-readable instructions" as used in the description and claims, include routines, applications, application modules, program modules, programs, components, data structures, algorithms, and the like. Computer-readable instructions can be implemented on various system configurations, including single-processor or multiprocessor systems, minicomputers, mainframe computers, personal computers, hand-held computing devices, microprocessor-based, programmable consumer electronics, combinations thereof, and the like.

The computer-readable storage media may include volatile memory (such as random-access memory ("RAM")) and/or non-volatile memory (such as read-only memory ("ROM"), flash memory, etc.). The computer-readable storage media may also include additional removable storage and/or non-removable storage including, but not limited to, flash memory, magnetic storage, optical storage, and/or tape storage that may provide non-volatile storage of computer-readable instructions, data structures, program modules, and the like.

A non-transient computer-readable storage medium is an example of computer-readable media. Computer-readable media includes at least two types of computer-readable media, namely computer-readable storage media and communications media. Computer-readable storage media includes volatile and non-volatile, removable and non-removable media implemented in any process or technology for storage of information such as computer-readable instructions, data structures, program modules, or other data. Computer-readable storage media includes, but is not limited to, phase change memory ("PRAM"), static random-access memory ("SRAM"), dynamic random-access memory ("DRAM"), other types of random-access memory ("RAM"), read-only memory ("ROM"), electrically erasable programmable read-only memory ("EEPROM"), flash memory or other memory technology, compact disk read-only memory ("CD-ROM"), digital versatile disks ("DVD") or other optical storage, magnetic cassettes, magnetic tape, magnetic disk storage or other magnetic storage devices, or any other non-transmission medium that can be used to store information for access by a computing device. In contrast, communication media may embody computer-readable instructions, data structures, program modules, or other data in a modulated data signal, such as a carrier wave, or other transmission mechanism. As defined herein, computer-readable storage media do not include communication media.

The computer-readable instructions stored on one or more non-transitory computer-readable storage media that, when executed by one or more processors, may perform operations described above with reference to FIGS. 1 and 2. Generally, computer-readable instructions include routines, programs, objects, components, data structures, and the like that perform particular functions or implement particular abstract data types. The order in which the operations are described is not intended to be construed as a limitation, and any number of the described operations can be combined in any order and/or in parallel to implement the processes.

## Claims

1. A system (300) comprising:
one or more processors (302); and
memory (304) communicatively coupled to the one or more processors, the memory storing computer-executable modules executable by the one or more processors that, when executed by the one or more processors, perform associated operations, the computer-executable modules (306) comprising:
a manual input collecting module (308) configured to collect manual input from an input interface (140, 340) of a pain assessment regularizing system (102);
a physiological measurement input collecting module (312) configured to collect physiological measurement input from a physiological sensor (108) of the pain assessment regularizing system affixed to an operative position on a patient's body;
an input weighing module (316) configured to weigh at least one of the manual input and the physiological measurement input;
an input converting module (318) configured to convert each of the manual input and the physiological measurement input into a respective regularized pain assessment factor;
a combining module (320) configured to derive a combined regularized pain assessment factor collectively from each regularized pain assessment factor;
a recording module (322) configured to record each combined regularized pain assessment factor in a time series;
a forecasting module (324) configured to forecast a trend of the time series of combined regularized pain assessment factors by fitting a regression model to the time series; and
a visualizing module (326) configured to display the time series and the trend on an output interface (114, 340).

2. The system of claim 1, wherein the computer-executable modules further comprise an image capture input collecting module (310) configured to collect image capture input or video capture input from an image capture device (106) of the pain assessment regularizing system directed upon an expressive location on a patient's body; and
wherein the input weighing module (316) is further configured to weigh the image capture input or video capture input; and
wherein the input converting module (318) is further configured to convert the image capture input or video capture input into a regularized pain assessment factor.

3. The system of claim 2, wherein the expressive location and the operative position do not correspond to a location where the patient is experiencing or reporting pain.

4. The system of any preceding claim, wherein the physiological measurement input comprises at least one of: blood pressure; pulse rate or pulse waveform; respiratory rate; galvanic skin response; cortical activity; muscle contraction; and blood carbon dioxide level.

5. The system of any preceding claim, wherein the computer-executable modules further comprise a patient-controlled analgesia input collecting module (314) configured to collect patient-controlled analgesia (PCA) input from a PCA device intravenously connected to a patient; and
wherein the input weighing module is further configured to weigh the PCA input; and
wherein the input converting module is further configured to convert the PCA input into a regularized pain assessment factor.

6. A computer-readable storage medium (304) storing computer-readable instructions executable by one or more processors (302), that when executed by the one or more processors, cause the one or more processors to perform operations comprising:
collecting manual input from an input interface (104) of a pain assessment regularizing system (102);
collecting physiological measurement input from a physiological sensor (108) of the pain assessment regularizing system affixed to an operative position on a patient's body;
weighing at least one of the manual input and the physiological measurement input;
converting each of the manual input and the physiological measurement input into a respective regularized pain assessment factor;
deriving a combined regularized pain assessment factor collectively from each regularized pain assessment factor;
recording each combined regularized pain assessment factor in a time series;
forecasting a trend of the time series of combined regularized pain assessment factors by fitting a regression model to the time series; and
displaying a visualization of the time series and the trend on an output interface.

7. The computer-readable storage medium of claim 6, wherein the operations further comprise:
collecting image capture input or video capture input from an image capture device (106) of the pain assessment regularizing system directed upon an expressive location on a patient's body;
weighing the image capture input or video capture input; and
converting the image capture input or video capture input into a respective regularized pain assessment factor.

8. The computer-readable storage medium of claim 7, wherein the expressive location and the operative position do not correspond to a location where the patient is experiencing or reporting pain.

9. The computer-readable storage medium of any one of claims 6 to 8, wherein the physiological measurement input comprises at least one of: blood pressure; pulse rate or pulse waveform; respiratory rate; galvanic skin response; cortical activity; and blood carbon dioxide level.

## Patentansprüche

1. System (300), umfassend:
einen oder mehrere Prozessoren (302); und
Speicher (304), der kommunikativ mit dem einen oder den mehreren Prozessoren gekoppelt ist, wobei der Speicher computerausführbare Module speichert, die von dem einen oder den mehreren Prozessoren ausführbar sind, die, wenn sie von dem einen oder den mehreren Prozessoren ausgeführt werden, zugehörige Vorgänge durchführen, wobei die computerausführbaren Module (306) Folgendes umfassen:
ein Erfassungsmodul für manuelle Eingaben (308), das konfiguriert ist, um manuelle Eingaben von einer Eingabeschnittstelle (140, 340) eines Schmerzbewertungsregularisierungssystems (102) zu erfassen;
ein Erfassungsmodul für Eingaben physiologischer Messung (312), das konfiguriert ist, um Eingaben einer physiologischen Messung von einem physiologischen Sensor (108) des Schmerzbewertungsregularisierungssystems zu erfassen, der an einer wirksamen Position am Körper eines Patienten angebracht ist;
ein Eingabegewichtungsmodul (316), das konfiguriert ist, um mindestens eine von der manuellen Eingabe und der Eingabe der physiologischen Messung zu gewichten;
ein Eingabeumwandlungsmodul (318), das konfiguriert ist, um jede von der manuellen Eingabe und der Eingabe der physiologischen Messung in einen jeweiligen regularisierten Schmerzbewertungsfaktor umzuwandeln;
ein Kombinierungsmodul (320), das konfiguriert ist, um einen kombinierten regularisierten Schmerzbewertungsfaktor gemeinsam von jedem regularisierten Schmerzbewertungsfaktor abzuleiten;
ein Aufzeichnungsmodul (322), das konfiguriert ist, um jeden kombinierten regularisierten Schmerzbewertungsfaktor in einer Zeitreihe aufzuzeichnen;
ein Vorhersagemodul (324), das konfiguriert ist, um einen Trend der Zeitreihe der kombinierten regularisierten Schmerzbewertungsfaktoren durch Anpassen eines Regressionsmodells an die Zeitreihe vorherzusagen; und
ein Visualisierungsmodul (326), das konfiguriert ist, um die Zeitreihe und den Trend auf einer Ausgabeschnittstelle (114, 340) anzuzeigen.

2. System nach Anspruch 1, wobei die computerausführbaren Module weiter ein Erfassungsmodul für Bildaufnahmeeingaben (310) umfassen, das konfiguriert ist, um Bildaufnahmeeingaben oder Videoaufnahmeeingaben von einer Bildaufnahmevorrichtung (106) des Schmerzbewertungsregularisierungssystems zu erfassen, die auf eine aussagefähige Stelle auf dem Körper eines Patienten gerichtet ist; und
wobei das Eingabegewichtungsmodul (316) weiter konfiguriert ist, um die Bildaufnahmeeingaben oder Videoaufnahmeeingaben zu gewichten; und
wobei das Eingabeumwandlungsmodul (318) weiter konfiguriert ist, um die Bildaufnahmeeingaben oder Videoaufnahmeeingaben in einen regularisierten Schmerzbewertungsfaktor umzuwandeln.

3. System nach Anspruch 2, wobei die aussagefähige Stelle und die wirksame Position nicht einer Stelle entsprechen, an der der Patient Schmerzen erfährt oder über Schmerzen berichtet.

4. System nach einem vorstehenden Anspruch, wobei die Eingabe der physiologischen Messung mindestens eines umfasst aus: Blutdruck; Pulsfrequenz oder Pulswellenform; Atemfrequenz; galvanischer Hautreaktion, kortikaler Aktivität; Muskelkontraktion; und Kohlendioxidspiegel im Blut.

5. System nach einem vorstehenden Anspruch, wobei die computerausführbaren Module weiter ein Erfassungsmodul für patientengesteuerte Analgesie-Eingaben (314) umfassen, das konfiguriert ist, um patientengesteuerte Analgesie- (PCA-) Eingaben von einer PCA-Vorrichtung, die intravenös mit einem Patienten verbunden ist, zu erfassen; und
wobei das Eingabegewichtungsmodul weiter konfiguriert ist, um die PCA-Eingabe zu gewichten; und
wobei das Eingabeumwandlungsmodul weiter konfiguriert ist, um die PCA-Eingabe in einen regularisierten Schmerzbewertungsfaktor umzuwandeln.

6. Computerlesbares Speichermedium (304), das computerlesbare Anweisungen speichert, die von einem oder mehreren Prozessoren (302) ausführbar sind, die, wenn sie von dem einen oder den mehreren Prozessoren ausgeführt werden, den einen oder die mehreren Prozessoren veranlassen, Vorgänge durchzuführen, umfassend:
Erfassen von manuellen Eingaben von einer Eingabeschnittstelle (104) eines Schmerzbewertungsregularisierungssystems (102);
Erfassen von Eingaben physiologischer Messung von einem physiologischen Sensor (108) des Schmerzbewertungsregularisierungssystems, der an einer wirksamen Position am Körper eines Patienten angebracht ist;
Gewichten mindestens einer von der manuellen Eingabe und der Eingabe der physiologischen Messung;
Umwandeln jeder von der manuellen Eingabe und der Eingabe der physiologischen Messung in einen jeweiligen regularisierten Schmerzbewertungsfaktor;
Ableiten eines kombinierten regularisierten Schmerzbewertungsfaktors gemeinsam von jedem regularisierten Schmerzbewertungsfaktor;
Aufzeichnen jedes kombinierten regularisierten Schmerzbewertungsfaktors in einer Zeitreihe;
Vorhersagen eines Trends der Zeitreihe der kombinierten regularisierten Schmerzbewertungsfaktoren durch Anpassen eines Regressionsmodells an die Zeitreihe; und
Anzeigen einer Visualisierung der Zeitreihe und des Trends auf einer Ausgabeschnittstelle.

7. Computerlesbares Speichermedium nach Anspruch 6, wobei die Vorgänge weiter umfassen:
Erfassen von Bildaufnahmeeingaben oder Videoaufnahmeeingaben von einer Bildaufnahmevorrichtung (106) des Schmerzbewertungsregularisierungssystems, die auf eine aussagefähige Stelle auf dem Körper eines Patienten gerichtet ist;
Gewichten der Bildaufnahmeeingaben oder Videoaufnahmeeingaben; und
Umwandeln der Bildaufnahmeeingaben oder Videoaufnahmeeingaben in einen jeweiligen regularisierten Schmerzbewertungsfaktor.

8. Computerlesbares Speichermedium nach Anspruch 7, wobei die aussagefähige Stelle und die wirksame Position nicht einer Stelle entsprechen, an der der Patient Schmerzen erfährt oder über Schmerzen berichtet.

9. Computerlesbares Speichermedium nach einem der Ansprüche 6 bis 8, wobei die Eingabe der physiologischen Messung mindestens eines umfasst aus: Blutdruck; Pulsfrequenz oder Pulswellenform; Atemfrequenz; galvanischer Hautreaktion, kortikaler Aktivität; und Kohlendioxidspiegel im Blut.

## Revendications

1. Système (300) comprenant :
un ou plusieurs processeurs (302) ; et
une mémoire (304) couplée en communication avec le un ou les plusieurs processeurs, la mémoire stockant des modules exécutables par ordinateur exécutables par le un ou les plusieurs processeurs qui, lorsqu'ils sont exécutés par le un ou les plusieurs processeurs, réalisent des opérations associées, les modules exécutables par ordinateur (306) comprenant :
un module de collecte d'intrant manuel (308) configuré pour collecter un intrant manuel à partir d'une interface d'intrant (140, 340) d'un système de régularisation d'évaluation de la douleur (102) ;
un module de collecte d'intrant de mesure physiologique (312) configuré pour collecter un intrant de mesure physiologique à partir d'un capteur physiologique (108) du système de régularisation d'évaluation de la douleur apposé dans une position de fonctionnement sur le corps d'un patient ;
un module de pondération d'intrant (316) configuré pour pondérer au moins un de l'intrant manuel et de l'intrant de mesure physiologique ;
un module de conversion d'intrant (318) configuré pour convertir chacun de l'intrant manuel et de l'intrant de mesure physiologique en un facteur d'évaluation de la douleur régularisé respectif ;
un module de combinaison (320) configuré pour dériver un facteur d'évaluation de la douleur régularisé combiné collectivement à partir de chaque facteur d'évaluation de la douleur régularisé ;
un module d'enregistrement (322) configuré pour enregistrer chaque facteur d'évaluation de la douleur régularisé combiné en une série chronologique ;
un module de prévision (324) configuré pour prévoir une tendance de la série chronologique de facteurs d'évaluation de la douleur régularisés combinés en ajustant un modèle de régression sur la série chronologique ; et
un module de visualisation (326) configuré pour afficher la série chronologique et la tendance sur une interface de sortie (114, 340).

2. Système selon la revendication 1, dans lequel les modules exécutables par ordinateur comprennent en outre un module de collecte d'intrant de capture d'image (310) configuré pour collecter un intrant de capture d'image ou un intrant de capture de vidéo à partir d'un dispositif de capture d'image (106) du système de régularisation d'évaluation de la douleur dirigé sur un emplacement expressif sur le corps d'un patient ; et
dans lequel le module de pondération d'intrant (316) est en outre configuré pour pondérer l'intrant de capture d'image ou l'intrant de capture de vidéo ; et
dans lequel le module de conversion d'intrant (318) est en outre configuré pour convertir l'intrant de capture d'image ou l'intrant de capture de vidéo en un facteur d'évaluation de la douleur régularisé.

3. Système selon la revendication 2, dans lequel l'emplacement expressif et la position de fonctionnement ne correspondent pas à un emplacement où le patient éprouve ou signale une douleur.

4. Système selon une quelconque revendication précédente, dans lequel l'intrant de mesure physiologique comprend au moins l'un des éléments suivants : pression artérielle ; fréquence de pouls ou forme d'onde de pouls ; fréquence respiratoire ; réflexe psychogalvanique ; activité corticale ; contraction musculaire ; et teneur en dioxyde de carbone dans le sang.

5. Système selon une quelconque revendication précédente, dans lequel les modules exécutables par ordinateur comprennent en outre un module de collecte d'intrant d'analgésie contrôlée par le patient (314) configuré pour collecter un intrant d'analgésie contrôlée par le patient (PCA) à partir d'un dispositif PCA connecté intraveineusement à un patient ; et
dans lequel le module de pondération d'intrant est en outre configuré pour pondérer l'intrant PCA ; et
dans lequel le module de conversion d'intrant est en outre configuré pour convertir l'intrant PCA en un facteur d'évaluation de la douleur régularisé.

6. Support de stockage lisible par ordinateur (304) stockant des instructions lisibles par ordinateur exécutables par un ou plusieurs processeurs (302), qui, lorsqu'elles sont exécutées par le un ou les plusieurs processeurs, amènent le un ou les plusieurs processeurs à réaliser des opérations comprenant :
la collecte d'un intrant manuel à partir d'une interface d'intrant (104) d'un système de régularisation d'évaluation de la douleur (102) ;
la collecte d'un intrant de mesure physiologique à partir d'un capteur physiologique (108) du système de régularisation d'évaluation de la douleur apposé dans une position de fonctionnement sur le corps d'un patient ;
la pondération d'au moins un de l'intrant manuel et de l'intrant de mesure physiologique ;
la conversion de chacun de l'intrant manuel et de l'intrant de mesure physiologique en un facteur d'évaluation de la douleur régularisé respectif ;
la dérivation d'un facteur d'évaluation de la douleur régularisé combiné collectivement à partir de chaque facteur d'évaluation de la douleur régularisé ;
l'enregistrement de chaque facteur d'évaluation de la douleur régularisé combiné en une série chronologique ;
la prévision d'une tendance de la série chronologique de facteurs d'évaluation de la douleur régularisés combinés en ajustant un modèle de régression sur la série chronologique ; et
l'affichage d'une visualisation de la série chronologique et de la tendance sur une interface de sortie.

7. Support de stockage lisible par ordinateur selon la revendication 6, dans lequel les opérations comprennent en outre :
la collecte d'un intrant de capture d'image ou d'un intrant de capture de vidéo à partir d'un dispositif de capture d'image (106) du système de régularisation d'évaluation de la douleur dirigé sur un emplacement expressif sur le corps d'un patient ;
la pondération de l'intrant de capture d'image ou de l'intrant de capture de vidéo ; et
la conversion de l'intrant de capture d'image ou de l'intrant de capture de vidéo en un facteur d'évaluation de la douleur régularisé respectif.

8. Support de stockage lisible par ordinateur selon la revendication 7, dans lequel l'emplacement expressif et la position de fonctionnement ne correspondent pas à un emplacement où le patient éprouve ou signale une douleur.

9. Support de stockage lisible par ordinateur selon l'une quelconque des revendications 6 à 8, dans lequel l'intrant de mesure physiologique comprend au moins l'un des éléments suivants : pression artérielle ; fréquence de pouls ou forme d'onde de pouls ; fréquence respiratoire ; réflexe psychogalvanique ; activité corticale ; et teneur en dioxyde de carbone dans le sang.
